# EUROPEAN PATENT SPECIFICATION

(11) **EP 3 384 880 B1**
(45) Date of publication and mention of the grant of the patent: **17.02.2021**
(21) Application number: 17164987.4
(22) Date of filing: 05.04.2017
(51) Int. Cl.: A61F 5/56, A61M 16/20

(54) **BUCCAL VALVE**
BUKKALVENTIL
SOUPAPE BUCCALE

(43) Date of publication of application: 10.10.2018
(73) Proprietor: rhinoloop SA, 1005 Lausanne (CH)
(72) Inventor: Martin Prieto, Antonio, 1207 Genève (CH)
(74) Representative: Temiño Ceniceros, Ignacio

(56) References cited:
- WO-A1-2009/149507
- US-A1- 2001 052 344
- US-A1- 2008 257 358
- US-A1- 2010 326 447
- US-A1- 2011 005 520
- US-B1- 8 261 748

## Description

### FIELD OF THE INVENTION

The present invention belongs to the valves that regulate and modify the respiratory flow. More specifically, the invention relates to a finned buccal valve to modify the respiratory flow of the user, the primary purpose of which is to create positive expiratory pressure (PEP) in the respiratory tract.

### BACKGROUND OF THE INVENTION

There is currently no buccal valve on the market with the features of the invention to be described herein.

There is a great variety of "buccal devices" for the modification of the respiratory flow, but the great majority of them cannot be classified as "valve", because they do not contain the constituent parts of the valve concept. Said parts are: the movable part (flap, ball, air passage, guillotine, etc.) and the fixed part which supports said movable part (valve body).

In general, the "valves" that exist in the market are usually added to the aforementioned buccal devices as an external element, which performs the functions the buccal device cannot do. For example, WO2009/149507 A1 discloses devices for inclusion in personal respiratory systems for use in unsafe ambient atmosphere, in order to provide a clean air to an individual in the form of a mouth-bit module, intended for permitting inhalation through the mouth only, to which a source of clean air is attached.

Most of the aforementioned buccal devices using different methods to facilitate and maximize the airflow to the respiratory system, but do not have the capacity to act during the exit of the airflow from the respiratory system. They delegate this responsibility to "valves" that are external to them and are not a constituent part of the invention of the buccal device.

These "valves" that are connected to the buccal devices do meet the requirements to be catalogued as valves or airflow resistors, however none of them can be classified as a "buccal valve", since they are not designed or intended to be used specifically in the buccal cavity.

The valve concept is a very old concept. By definition, all valves comprise at least a fixed part that may be called valve body and a movable part that can be called a valve flap/ball/passage etc., depending on its shape and functional logic. For instance, in US2008/257358 A1 a valve for treating sleep conditions is disclosed, which comprises a mouthpiece that pressurizes exhaled air to increase the pressure in the airway of an individual during the rest portion of the respiratocy cycle and maintaining the airway at least partially dilated through the entire respiratory cycle. Another case are devices based on altering the flow of air in a respiratory cavity (mouth, nostrils of the nose, etc.) so that it may selectively increase the resistance to expiration with regard to inspiration, as explained in US2011/005520 A1 and US2001/0052344 A1. Their goal is mimicking the pursed lip breathing, which is convenient for patients suffering from pulmonary disease, sleep apnea, etc. Further, US 8261748 B1 discloses a removable oral device for treating snoring and apnea, composed by a tongue attachment member along with a maxilla attachment. However, these devices suffer from certain limitations, such as the need of optimizing the contact between the valve flap and the valve body to better adapt said valve flap to different geometries of the valve body. Furthermore, it is required to improve the functionality of the valve flap with regard to its speed reaction in front of the change of airflow direction during the respiration cycle. Note also that the replacement of a damaged flap is difficult in the aforementioned devices, so this problem needs to be addressed.

Since this is an old concept, there are infinite types of valves with multiple applications in the market, but none of these have the features of the buccal valve of the invention described herein.

Some examples of airflow control valve inventions can be found in the following patent application references: US201615275419 (2016/09/25), CA20152947123 (2015/04/27), US201415024163 (2014/09/22), US201514813422 (2015/07/30), US201614989400 (2016/01/06), HK20120105659 (2012/06/11), US201213662907 (2012/10/29), AU20160277663 (2016/12/22), CA20152943913 (2015/03/26).

The previously mentioned buccal devices (non-valves), that already exist in the market may be catalogued according to the way they act on the anatomy of the user to modify the respiratory flow, for example: Tongue repositioning device (TRD); Repositioning the mandible device (MAD); Acting on the soft palate (ASPL); or using any type of existing valve or motor to create a positive oral pressure (OPAP).

Some illustrative examples of such buccal devices (non-valves) are: TRD (Tongue Retaining Device®); TLD (Tongue Locking Device®); TOPS (Tepper Oral Propioceptive Stimulator®); NAPA (Nocturnal Airway Patency Appliance®); Snore Guard®; Herbst; Jasper Jumper; IST-Herner (Intraoral Snoring Treatment®); SNOAR (Sleep and Nocturnal Obstructive Apnea Reducer®); SAS de Zurich®; Bionator; Twin-Block; Klearway®; Silencer®; Silensor (Silent Nite®); P.P.P. (Pistas Posteriores Planas); ASPL (Adjustable Soft Palate Lifter®); Equalizer (Equalizer Airway Device®); CPAP / biPAP/ nPAP.

An internal oral device that can be catalogued as a buccal valve is presently not known in the state of the art.

The buccal valve of the present invention is designed to work specifically within a user's buccal cavity proper, with the primary objective of creating positive expiratory pressure (PEP) in his/her respiratory tract.

The level of positive expiratory pressure (PEP) generated by the invention may depend either partially or totally on whether said buccal valve works in collaboration with some type of nasal device for the modification of respiratory flow, such as a toroidal nasal device. Other devices based on inducing PEP are known in the field, see US2010/326467 A1.

The present invention proposes, but is not limited to, a "buccal valve" designed to function in the buccal cavity of a user, and with respect to other valves of the state of the art it is characterized by the fact that it may simultaneously control the volume and direction of airflow entering the buccal cavity, reorient said flow inside the oral cavity depending on the position of the tongue and use the elastic properties of the soft tissues of the buccal anatomy to generate positive expiratory pressure (PEP) in the respiratory tract.

The positive expiratory pressure (PEP) generated by the buccal valve of the invention may be oriented towards the medical treatment of respiratory disorders, as well as to non-medical purposes such as sports training and activities with aerobic demands.

The technical accomplishment of the present invention thus makes it possible to overcome the above detailed problems of the valves and buccal devices of the state of the art.

### BRIEF DESCRIPTION OF THE INVENTION

The object of the present invention makes reference, though without limitation, to the development of a buccal valve, able to modify the respiratory flow within the buccal cavity of a user and comprising the features of claim 1.

In this document, the term "oral cavity proper" refers to the space of the buccal anatomy wherein the tongue is located, on the inside of the dental arches.

In this way, it is possible to attribute new functional capabilities to the constituent elements that define the valve flap concept, its movable element (flap or flaps) and its fixed element (the valve body).

The functional capabilities of the fundamental elements of the invention described herein, in its simplest version, are preferentially:
- For the fixed element (the valve body):
   - provide adequate support for the valve flap
   - control the passage of airflow entering the buccal cavity proper
   - control the direction of the airflow exiting the buccal cavity
- For the movable element (flap or flaps):
   - control simultaneously both the volume and direction of airflow entering the buccal cavity proper
   - reorient incoming airflow into the buccal cavity proper, depending on the position of the user's tongue
   - control the direction of airflow exiting the buccal cavity

The combination of such capabilities of the constituent parts of the buccal valve invention herein, characterizes it with respect to other valves of the state of the art that are already in the market.

In other technical embodiments of the invention, the buccal valve may also have the capacity to increase the level of resistance offered to the airflow exiting the buccal cavity during the expiratory phase, and the capacity to partially control the movement of the tongue within the buccal cavity.

The buccal valve of the invention described herein, in its simplest variant, does not control the level of resistance of the airflow exiting the buccal cavity of the user during the expiratory phase. This level of resistance will depend on the physical and anatomical features (shape, elasticity, density, roughness) specific of the soft tissues of the buccal anatomy of each user.

The primary objective of the invention described herein, in its simplest version, is to create a positive expiratory pressure in the airway of a user, forcing the outgoing airflow during the expiratory phase to exit through the deepest part of the buccal cavity through the ends of the valve body, and traverse the path between the dental arches and the cheek muscles before it can leave through the lips.

These soft and elastic tissues of the cheek, such as the buccinator muscle, block the only escape route of the airflow and provide resistance to the exit of the air. This causes an increase in the internal pressure (positive pressure) in the respiratory tract of the user.

In some variants, the buccal valve may increase the level of resistance offered to the airflow exiting the buccal cavity by connecting an additional element to its valve body (airflow resistance) which has the capacity to work together with the soft tissues of the buccal anatomy to achieve this end. Said element takes advantage of the physical features of these tissues (elasticity, viscosity, shape) to combine them with its own features (elasticity, shape, etc.) and generate a greater level of resistance to the airflow exiting the buccal cavity. This increment in resistance automatically generates an increase of pressure.

The positive expiratory pressure created by the invention described herein may be aimed at the medical treatment of different respiratory-type dysfunctions, although it may also be applied to non-therapeutic uses, such as sports activities and trainings or activities with aerobic demands.

Therefore, in the different embodiments of the invention described herein, facilitating the airflow entry through the buccal cavity into the respiratory system of the user is a secondary objective; it may even become a minimum airflow, which complies with the safety standards necessary to ensure the health of the user.

The achievements of the invention described herein are not intended to maximize, by all means, the airflow entering through the buccal cavity, but to be able to control the percentage of airflow entering the buccal cavity with respect to the percentage entering through the nasal cavity, so as to find an optimal balance between the two.

In order to achieve these objectives, both the primary and the secondary, some of the variants of the invention described herein may be designed to be able to work together with nasal devices that modify the respiratory flow, such as toroidal nasal devices and other known treatments.

In the various embodiments of the invention, the buccal valve may be configured to fit, either partially or totally, into the buccal cavity of a user and modify his/her respiratory flow, facilitating to a greater or lesser extent the air intake during the inspiratory phase, as well as creating a higher or lower level of positive expiratory pressure in the respiratory tract of the user during the expiratory phase.

The level of the positive expiratory pressure generated by the invention described herein may depend, either partially or totally, on whether said invention works simultaneously with other types of nasal devices to modify the respiratory flow.

All the different variants of the invention described herein may be configured with flexible and/or elastic elements, so as to be able to better adapt and adjust to the great variety of existing physiognomies.

The embodiments of the invention described herein control the volume and direction of the airflow entering the buccal cavity proper, and also reorient said airflow according to the position of the tongue through their movable element (flap), which has the features and the type of anchoring on the valve body that allows it to perform said function.

The invention described herein controls the direction of airflow entering the buccal cavity proper, in order to orient it towards areas of the buccal anatomy that favour the increase of the level of turbulence therein, and therefore lengthens the time of exposure of said flow to the moist tissues of the buccal cavity before arriving at the area of the pharynx.

This extended exposure time of the incoming airflow to the moist tissues of the buccal anatomy allows to purify and humidify said flow and increase its temperature, thereby reducing the risk of irritation of the body tissues in the area of the pharynx, trachea and lungs.

As previously mentioned, the invention described herein also controls the volume of airflow entering through the buccal cavity into the respiratory system, so that when mixed with the more humid airflow coming from the nasal cavity, the overall percentage of moisture will reduce the risk of irritation of the body tissues of the respiratory system.

The control of said incoming airflow volume is performed by adjusting the system anchoring the valve flap to the valve body, which allows it to increase or reduce the level of resistance offered to the airflow intake of the buccal cavity proper.

In all the variants of the invention described herein, the shape, type of anchoring and position in the buccal cavity of its movable part(s) (flap/flaps) also give it the capacity to reorient the airflow entering the buccal cavity proper towards the different areas thereof, depending on the position of the user's tongue.

So, depending on the position of the user (lying on the side, face up, face down, standing), the tongue will adopt a position that will influence the movable part of the invention (flap/s) and this will direct the incoming airflow towards one or several areas of the buccal anatomy (palatine, dental arch or sublingual area).

The correct functioning of the invention described herein depends on the combination of the previously mentioned features and capabilities of its two constituent and defining elements, its fixed element (valve body) and its movable element (valve flap).

In the examples described herein, the valve body may include one or several areas of its geometry, shaped so as to be able to receive an additional element (airflow resistance) that works in collaboration with the soft tissues of the buccal anatomy to partially control the level of resistance offered to the airflow exiting the oral cavity during the expiratory phase.

In other variants of the invention, the additional airflow resistance element is temporarily anchored to the valve body, so that, in the event of breakage or malfunction, it may be replaced by another element in good condition.

In the present invention, the airflow resistance includes a ball joint.

In other variants of the invention, the connection between the airflow resistance element and the valve body allows the distance between both to be adjusted.

In another preferred embodiment of the invention, the valve body and the additional airflow resistance element are fused to become a single unitary object.

In one of the embodiments of the invention described herein, the valve body is temporarily fixed by superficial adhesion to the upper and/or lower dental arch, adapting its own geometry to the shape of said dental arches.

In one of the variants of the invention described herein, the valve body is temporarily fixed by superficial adhesion to the upper and/or lower dental arch by a second material, that is installed between the valve body and the dental arches.

In either of the two previously mentioned fixing modes, the valve body may be temporarily fixed to the upper and/or lower dental arch of the buccal cavity, so that said valve body prevents, in the area where it is installed, the passage of airflow through the dental arches, except for the passage(s) of air foreseen for this purpose which pass through said valve body.

In another preferential embodiment of the invention the valve body includes a ball joint.

In another example of the invention, the valve flap has ridges in some areas of its geometry which reduce the contact surface between said flap and the valve body, in order to avoid excessive adhesion between the two objects that could be generated by a liquid interposed between them, and that would harm its correct functioning.

In the present invention the valve flap includes a ball joint.

In other embodiments of the invention, the connection between the valve flap and the valve body allows the distance between both to be adjusted.

In another of the embodiments of the invention described herein, the surfaces of the flap valve and the valve body that are exposed to contact with the tongue of the user are aligned in a curved plane within the buccal cavity proper. This generates a surface without jumps or ridges that improves the contact with the tongue and prevents possible lesions thereof during extended periods of use of the invention.

In other variants of the invention, the valve flap is temporarily anchored to the valve body, so that, in the event of breakage or malfunction, it may be replaced by another valve flap in good condition.

In another example of the invention described herein, the valve body includes one or more areas of its geometry shaped to receive an additional element that connects the two ends of the valve body, passing through the interior of the buccal cavity proper, in order to partially control the movement of the tongue towards the palatine and glossopharyngeal areas.

Said additional element, which is connected to the valve body and partially controls the movement of the tongue, may be made of either a rigid or flexible material depending on the mechanical requirements for the correct functioning of the invention.

In other examples of the invention, the element that controls the movement of the tongue is temporarily anchored to the valve body, so that, in the event of breakage or malfunction, it may be replaced by another element in good condition.

The combination of the functional capabilities previously mentioned in this document, attributed to the two constituent and defining parts of the invention described herein, is what characterizes the finned buccal valve compared to other devices of the state of the art.

For the use of the buccal valve, according to the present invention and after having defined, preferentially together with the competent medical authority, which are the additional elements that will constitute it, the user may install it in the buccal cavity with his/her own hands and breathe normally.

Once the valve has been installed and fixed in the buccal cavity, in the basic variant thereof, the user will immediately notice that the valve controls the volume and direction of the airflow entering the buccal cavity proper, as well as the direction and course of the airflow exiting the buccal cavity, increasing the pressure inside the user's respiratory tract.

Depending on the type of valve that the user has installed in his/her buccal cavity, the invention may control to a greater or lesser extent the previously mentioned parameters, such as the level of resistance encountered at the exit of the airflow and the movement of the tongue.

Once the invention has been removed from the buccal cavity, the user will be able to clean and disinfect the buccal valve with some liquid or element qualified for that purpose.

In another preferred example of the invention, at least one valve flap and/or the valve body are made of a flexible material, enabling it to better adapt and adjust to the wide variety of existing physiognomies of its users.

In another preferred example of the invention, the buccal valve is suitable for use in the treatment of diseases, respiratory disorders and/or sports training.

In another preferred example of the invention, at least a portion of the manufacturing materials are biocompatible and/or biodegradable.

The main object of the invention is, therefore, a buccal valve according to any of the claims described herein.

Following we will proceed to describe some of the preferred embodiments of the buccal valves, according to the invention, describing its main components as well as its installation process and arrangement in the different buccal anatomies.

### DESCRIPTION OF THE FIGURES

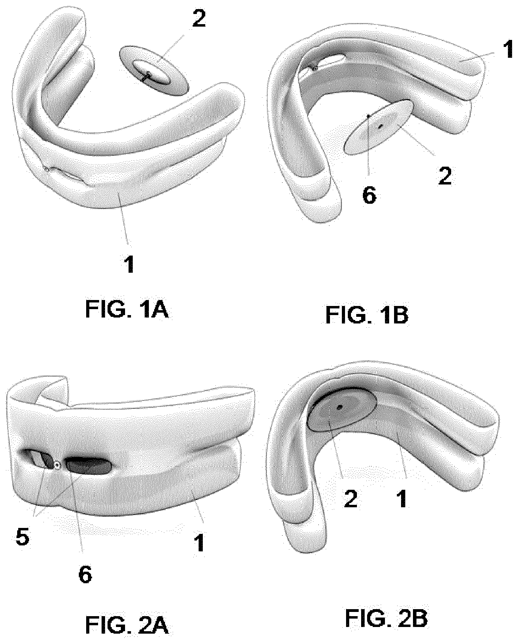

According to a preferred example of the invention, Figures 1A-1B show a detailed view of the buccal valve comprising a valve body and a valve flap.
According to a preferred example of the invention, Figures 2A-2B show a perspective view of the buccal valve comprising a valve body and a valve flap.
According to a preferred example of the invention, Figures 3A-3C show a cross-sectional and longitudinal view of the buccal valve.
Figure 4 shows a scheme of the buccal valve installed in the buccal cavity proper of the user, with the valve flap lodged in the buccal cavity proper exposed to contact with the tongue, according to a preferred example of the invention.
Figures 5A-5B show a diagram of the airflow path during the inspiratory and expiratory phases of the user when the buccal valve is installed in his/her buccal cavity, according to a preferred example of the invention. The buccal valve forces the airflow exiting during the expiratory phase from the buccal cavity to pass through the deepest part thereof, where the ends of the valve body are located.
Figures 6A-6B show a detailed perspective view of the buccal valve, further comprising an additional airflow resistance element according to a preferred example of the invention. Said additional element, together with the soft tissues of the anatomy of the face, gives the valve the capacity to increase the level of resistance offered by the airflow leaving the buccal cavity during the expiratory phase.
Figures 7A-7B show a perspective view of the buccal valve further comprising an additional airflow resistance element according to a preferred example of the invention.
Figures 8A-8C show a cross-sectional and longitudinal view of the buccal valve according to a preferred example of the invention.
Figure 9 shows a drawing of the buccal valve installed in the oral cavity of the user, further comprising an additional airflow resistance element lodged between the dental arches and the soft tissues of the face anatomy of the user, according to a preferred example of the invention.
Figure 10 shows a diagram of the airflow path during the inspiratory and expiratory phases of the user when the buccal valve with airflow resistance element is installed in his/her buccal cavity according to a preferred example of the invention. It shows how the buccal valve conducts the outgoing airflow to pass over the edge of the airflow resistance element, which is in contact with the soft tissues of the anatomy of the face.
Figure 11 shows a drawing of the buccal valve installed in the buccal cavity of the user, further comprising an additional airflow resistance element and a tongue movement controller, respectively controlling both the level of resistance offered at the exit of the airflow from the buccal cavity, and partially the movement of the tongue in the buccal cavity, according to a preferred example of the invention.
Figures 12A-12C show a cross-sectional and longitudinal view of the buccal valve installed in the buccal cavity of the user, further comprising an additional airflow resistance element and a tongue movement controller, according to a preferred example of the invention.
Figure 13, in a lateral cross-sectional view, shows a drawing of the buccal valve installed in the buccal cavity of the user, further comprising an additional airflow resistance element and a tongue movement controller, according to a preferred example of the invention. It shows that the tongue movement controller is connected to the ends of the valve body, passing through the buccal cavity and remaining at the top of the tongue.
Figures 14A and 14B show images of the valve body according to a preferred example of the invention, where we may see how the valve body helps to stabilize the valve in the buccal cavity, taking the shape of the dental arches and providing an adequate support to the valve flap and the additional elements.
Figures 15A-15D show several section planes of the valve body according to a preferred example of the invention, which show that the anchoring zone of the valve flap and the additional elements are located in the front part of the valve body between the central incisors of the dental arches.
Figures 16A-16B show a longitudinal cross-sectional view of the buccal valve with airflow resistance element according to a preferred example of the invention. It shows the shape of said resistance element, which may vary sufficiently to be anchored in the front area of the dental arches (the area of the incisors and canines), and provide adequate support for the valve flap.
Figures 17A-17B show a drawing indicating how the valve body of the invention may be attached to the dental arches, according to a preferred example of the invention. The valve body may be affixed to said arches either adapting its own geometry to the shape thereof or adopting a geometry that allows to interpose an intermediate element manufactured in a mouldable material that takes the shape of said arches and of the valve body.
Figure 18 shows in detail the different sections that the valve body may take, according to a preferred example of the invention, to adapt to the different positions of the user's dental arches.
Figures 19A-19B show in detail the valve flap according to a preferred example of the invention, indicating its sunshade-like shape and its anchoring system with the valve body.
Figure 20 shows sections of the valve flap in accordance with a preferred example of the invention. The valve flap connects to the valve body through an anchoring arm and a ball joint that allows for a relative rotation of the valve flap.
Figure 21 in a lateral view shows a drawing of the buccal valve installed in the buccal cavity of the user, according to a preferred example of the invention. It shows how the valve flap can regulate the volume and direction of the airflow entering the buccal cavity.
Figures 22A-22B show a drawing of the valve flap according to a preferred example of the invention. The valve flap may reorient the airflow entering the buccal cavity proper, depending on the position of the user's tongue when he/she is face up, standing, and lying on the left or on the right side.
Figure 23 shows a detail of the buccal valve according to a preferred example of the invention. The valve flap can adapt its angle and distance with respect to the valve body by adjusting its anchoring with the valve body and the ball joint connection between the elements that make up the valve flap, in order to control the contact surface between the flap, the valve and the resistance offered by the flap at the entry of the airflow into the buccal cavity.
Figure 24 shows a detail of the contact zone between the valve flap and the valve body according to a preferred example of the invention, showing the alignment between the surface of the valve flap and the surface of the valve body, when both are exposed to contact with the user's tongue.
Figure 25 shows in detail the valve flap according to a preferred example of the invention, where some ridges (10) may be seen on the surface of the valve flap, which reduce the contact surface between said flap (2) and the valve body (1), in order to prevent excessive adhesion between them that could be generated by a liquid interposed between them, which could damage the correct functioning of said flap (2).
Figure 26 shows the possible sections of the valve flap according to a preferred example of the invention, showing the different shapes that the flap may take to adapt to the varied buccal physiognomies of the users.
Figures 27A-27B show in detail the additional airflow resistance element, according to a preferred example of the invention.
Figure 28 shows in detail the additional airflow resistance element according to a preferred example of the invention, indicating the ball joint connection between the component elements and the anchor arm to connect said airflow resistance element with the valve body.
Figures 29A-29B show a longitudinal section of the buccal valve, according to a preferred example of the invention.
Figure 30 shows a detail of the contact area between the edge of the outer perimeter of the airflow resistance element and the soft tissues of the face anatomy, showing the equal and opposing forces respectively exerted, increasing the contact surface between them, and the path the outgoing airflow must take during the expiratory phase to pass through said contact area.
Figure 31 shows in detail the tongue movement controller of the buccal valve, according to a preferred example of the invention, where it is possible to observe the longitudinal main structure which can be connected to the valve body, and the superficial support element covering said main longitudinal structure that is designed to rest on top of the user's tongue.
Figure 32 shows in detail a longitudinal section of the tongue movement controller of the buccal valve according to a preferred example of the invention. It is composed of an elastic longitudinal main structure that fixes the controller to the valve body, and a superficial support element that covers the main structure without adhering thereto; said tongue movement controller is designed to rest on top of the tongue in order to partially control its movement towards the palatine and glossopharyngeal area.
Figure 33 shows the tongue movement controller element according to a preferred example of the invention. It may be elastically deformed to fit the perimeter of the tongue within the buccal cavity.
Figure 34 shows a detail of the type of anchoring between the tongue movement controller and the valve body, according to a preferred example of the invention. It shows the rotation of a part of the anchoring to keep the tongue movement controller and the valve body connected.
Figures 35A-35C show the cross-sections of the different tongue movement controllers, according to different preferred examples of the invention, in order to improve their adherence with the tongue and comfort of use.

### NUMERICAL REFERENCES USED IN THE FIGURES

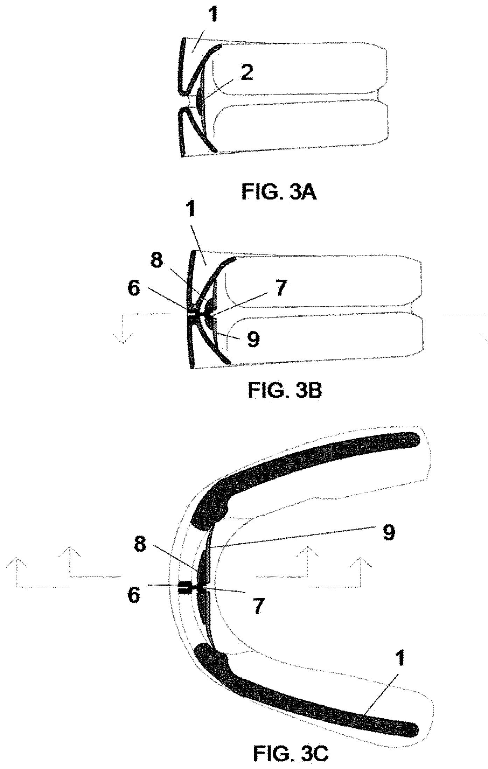

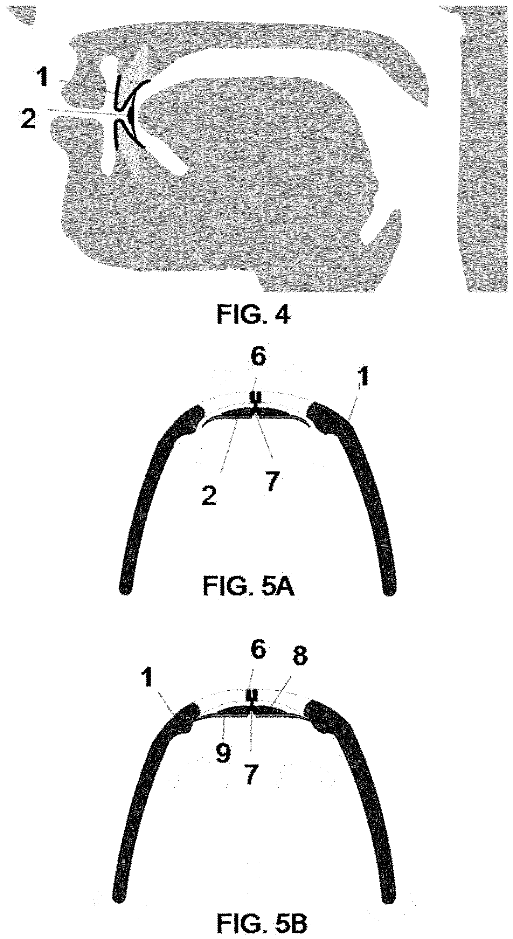

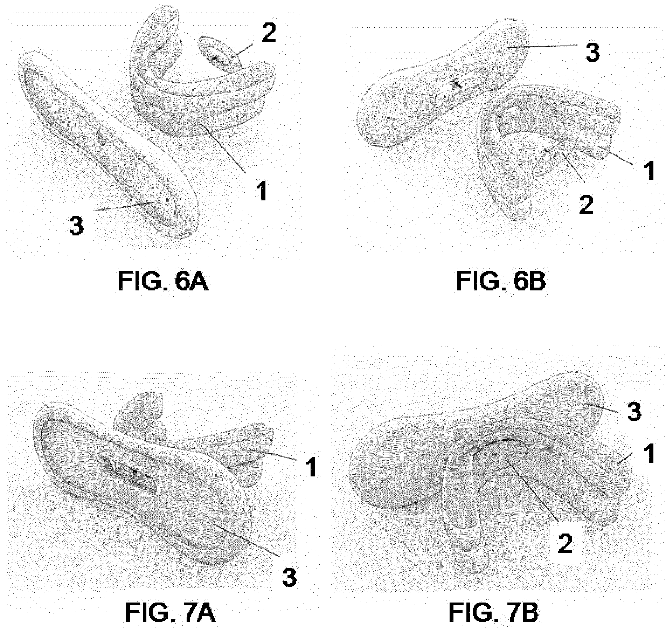

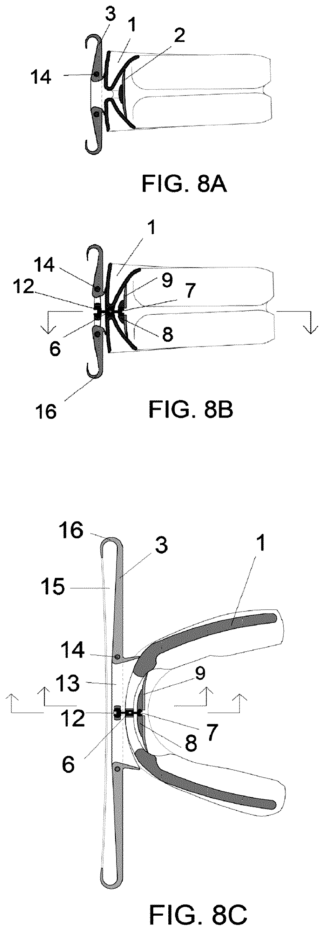

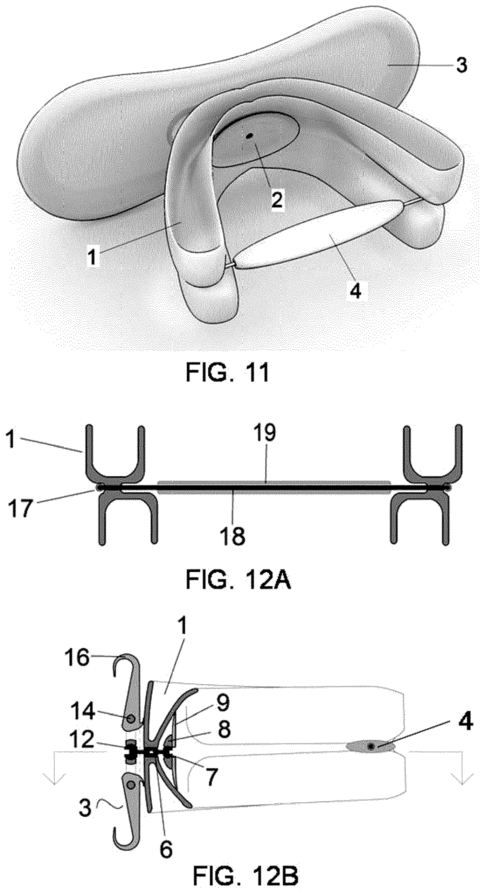

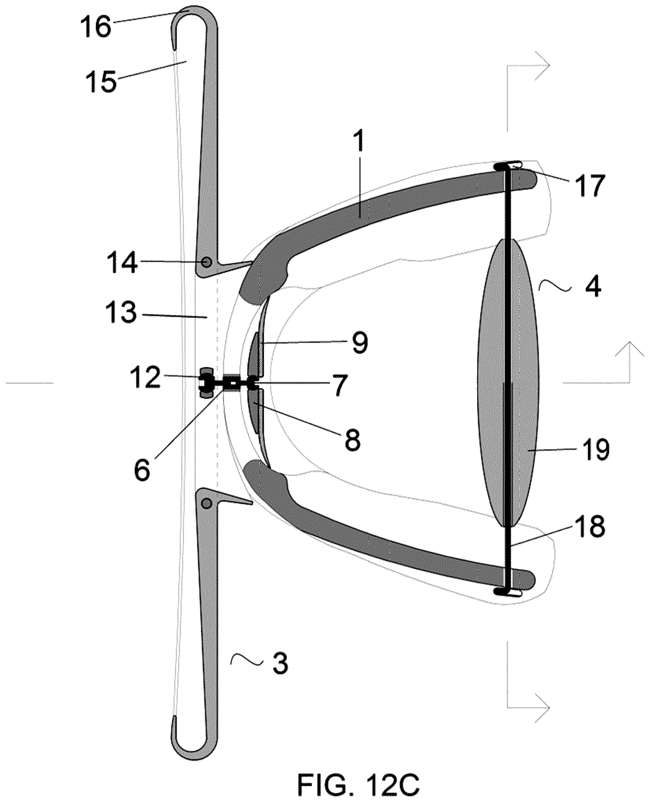

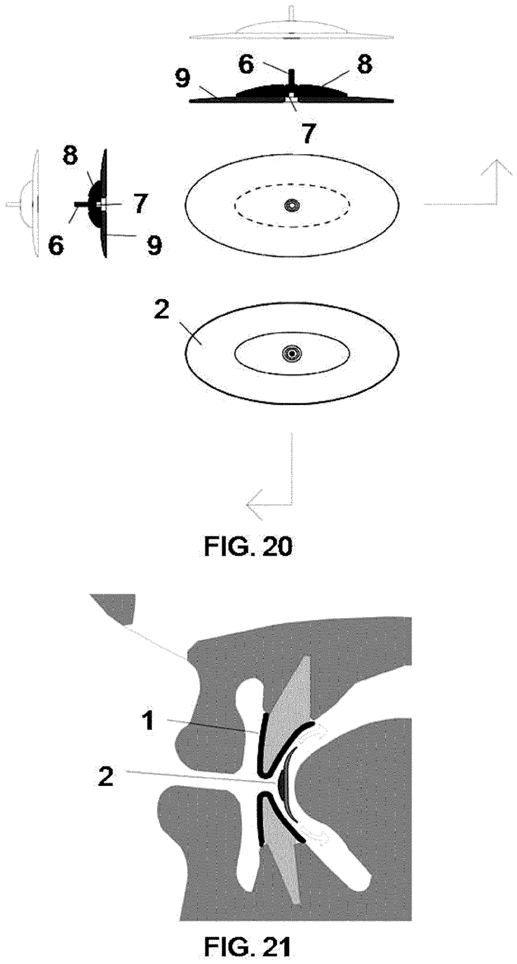

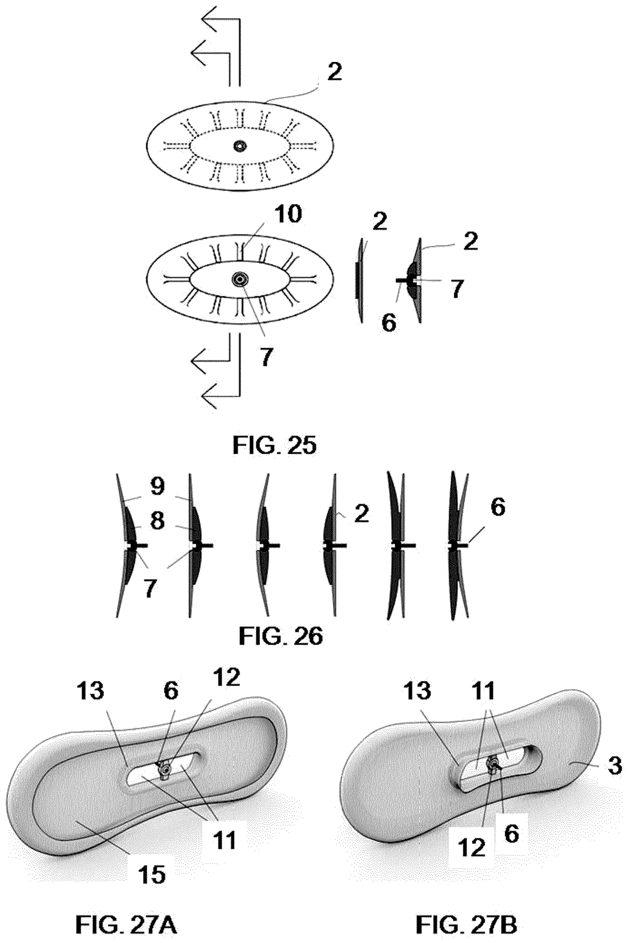

In order to help a better understanding of the technical features of the invention, Figures 1-35 are accompanied by a series of numerical references where, with an illustrative and non-limiting character, the following is represented:

| | |
|---|---|
| (1) | Valve body |
| (2) | Valve flap |
| (3) | Airflow resistance element |
| (4) | Tongue movement controller |
| (5) | Airflow passage of the valve body |
| (6) | Zone/anchor arm of the valve body |
| (7) | Valve flap ball joint |
| (8) | Flap membrane support |
| (9) | Flap membrane |
| (10) | Flap ridges |
| (11) | Airflow passages of the airflow resistance element |
| (12) | Zone/anchor arm/ball joint of the airflow resistance |
| (13) | Airflow resistance membrane |
| (14) | Airflow resistance membrane support |
| (15) | Outer front edge of the airflow resistance membrane |
| (16) | Internal curved outer edge of the airflow resistance membrane |
| (17) | Anchoring of the tongue controller |
| (18) | Longitudinal main structure of the tongue controller |
| (19) | Superficial support element of the tongue movement controller |
| (20) | Ridges of the tongue movement controller |

### DETAILED DESCRIPTION OF THE INVENTION

We then proceed to describe a preferred example of the present invention, provided with illustrative but not limitative purposes thereof.

A main object of the invention refers, as described in the preceding paragraphs and as shown in Figures 1-35 of the present document, to a buccal valve suitable to modify the respiratory flow of a user.

### - Main elements of the buccal valve according to the invention:

The buccal valve, according to the invention, is composed of two essential elements, with a series of specific functional capabilities that define and constitute it; the fixed part or valve body (1) and the movable part or valve flap (2). Without either of these parts, the concept of valve would not exist.

In some of the variants of the invention described herein, the buccal valve incorporates a series of additional elements that may be connected to its valve body (1), such as an airflow resistance element (3) and a tongue movement controller (4), which allow it to perform new functions, and are added to the functions of the constituent elements.

The functional capabilities that characterize the buccal valve of the invention in its most basic variant, with regard to other valves of the state of the art, are essentially: the capacity to simultaneously control the volume and direction of the airflow entering the buccal cavity proper and the capacity to reorient the airflow entering into the buccal cavity proper in terms of the position of the user's tongue.

The valve flap (2) of the invention has these new capabilities by means of: its geometry, its mechanical capabilities, the way it connects with the valve body (1) and its position with respect to the buccal anatomy of the user within the buccal cavity proper and exposed to direct contact with the tongue.

In a preferred example of the invention, the functional capabilities of the valve body (1) can be: control the airflow through the dental arches, control the direction of outgoing airflow during exhalation, receive one or several additional external elements and provide an adequate support for the correct functioning of the valve flap (2).

The valve body (1) of the invention described herein may acquire the previously described functional capabilities by adjusting its own geometry and adapting the materials that compose it to meet the mechanical conditions required by said functions.

In the different variants of the invention described herein, the physical and geometrical features of the additional elements, that are connected to the valve body (1) will determine their degree or level of performance in the functions accomplished by each of said elements, such as the level of resistance offered to the airflow exiting the buccal cavity, and control the movement of the tongue within the buccal cavity proper.

In the following paragraphs we will analyze in detail the possible physical and geometrical features of each of the elements of the different variants of the invention described herein, as well as the possible way of manufacturing them.

### - Valve body (1):

The valve body (1) is one of the two constituent and defining elements of the buccal valve invention described herein.

The valve body (1) of the invention described herein may have a great variety of geometrical shapes that may vary depending on the function to be performed in the different areas of the buccal anatomy of the user.

In order to meet the function of fixing the valve inside the buccal cavity, some preferred examples of the valve body (1) of the invention adopt a geometry that may be roughly approximated to the shape of the dental arches and allows it to fit in said arcs.

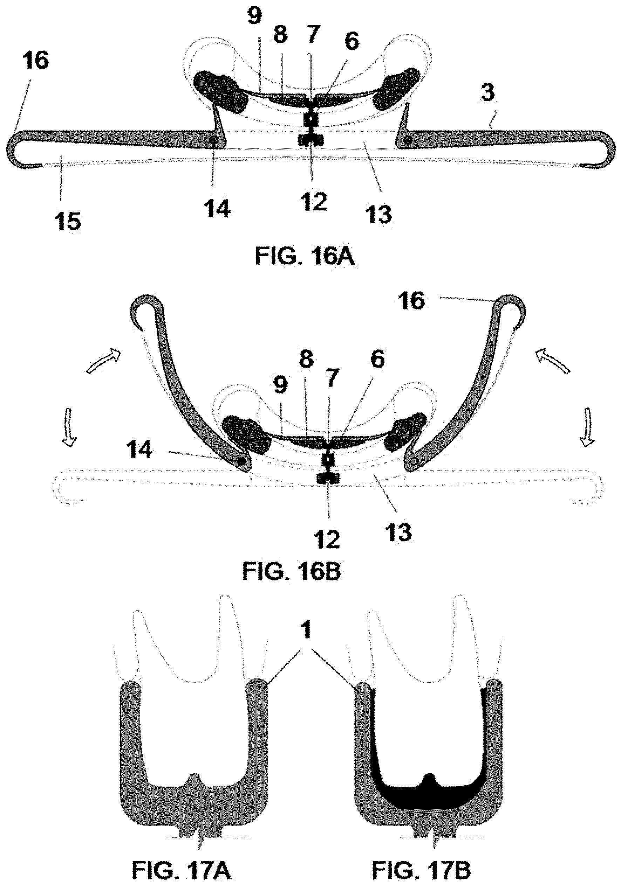

In a preferred example of the invention, the valve body (1) adapts its geometry to the exact shape of the dental arches into which it must fit (Fig. 17A) to be temporarily attached to them by superficial adherence.

In another preferred example of the invention, the valve body (1) adjusts its geometry to the approximate shape of the dental arches into which it should fit (Fig. 17B), leaving a space between the same and the teeth of the dental arch, in order to introduce a second material between them, which takes the exact shape of the dental arches and of the valve body (1), and which may temporarily fix the valve body (1) to said dental arches.

In another example of a buccal valve with airflow resistance element (3) (Figs. 16A-16B), it is seen how the valve body (1) adopts a minimum shape that allows it to be anchored in the front area of the dental arches (canines and incisors), provide an adequate support for contact with the valve flap (2) and to connect with the airflow resistance element.

In a preferred example of the invention, the valve body (1) comprises one or more airflow passages (5) incorporated in its geometry that allow the entry of airflow into the buccal cavity proper through the dental arches.

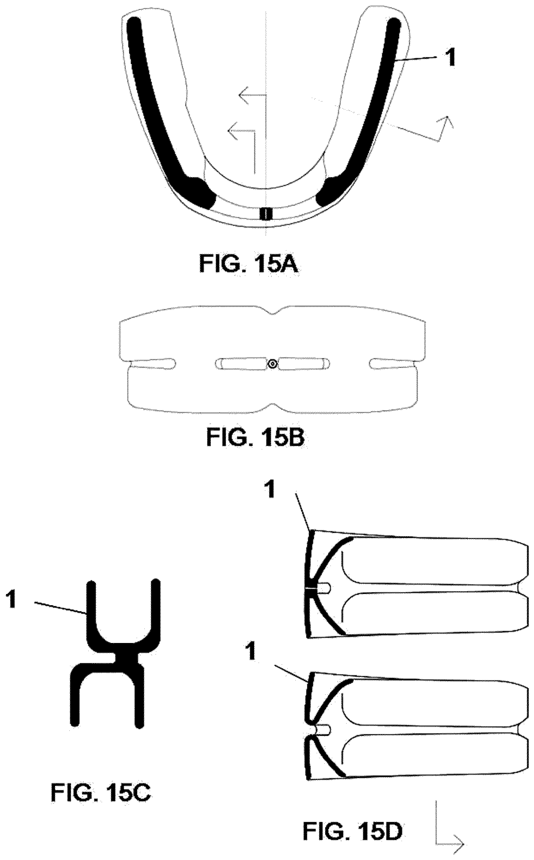

In order to meet the function of controlling the airflow passing through the dental arches area where the buccal valve is installed, in some preferred examples of the invention the valve body (1) adopts the shape of a continuous object (Fig.15) that connects with the dental arches and prevents the passage of airflow therethrough, except in the airflow passages (5) provided for therein, which pass through it.

The valve body (1) of the invention described herein may have one or more airflow passages incorporated in its geometry that allow the airflow to enter into the buccal cavity proper through the dental arches.

In the most basic preferred example of the invention (Fig. 1-2), the capacity to control the flow of air through the dental arches of the valve body (1) also enables it, working in collaboration with the valve flap (2), to control the direction of the airflow exiting the buccal cavity during exhalation, forcing said airflow out of the deeper part of the buccal cavity, where the ends of the valve body (1) are located.

The valve body (1), according to the invention described herein, may form one or several areas of its geometry to connect it with one or several external elements thereto, which give the buccal valve the capacity to perform a series of new functions.

In another of the preferred examples of the invention, the valve body (1) incorporates in its geometry an anchoring area (6), suitable to temporarily receive one or several elements external to it, in such a way that said connection allows to modify the distance between the valve body (1) and said external element. Said element is located at the front of the geometry of the valve body (1) between the central incisors of the user's dental arches.

In another preferred example of the invention, the valve body (1) adapts its geometry so that the concave surface generated by said valve body (1) inside the buccal cavity proper and on the inner side of the dental arches is as smooth and continuous as possible, and suitable for being in contact with the valve flap (2) and the user's tongue.

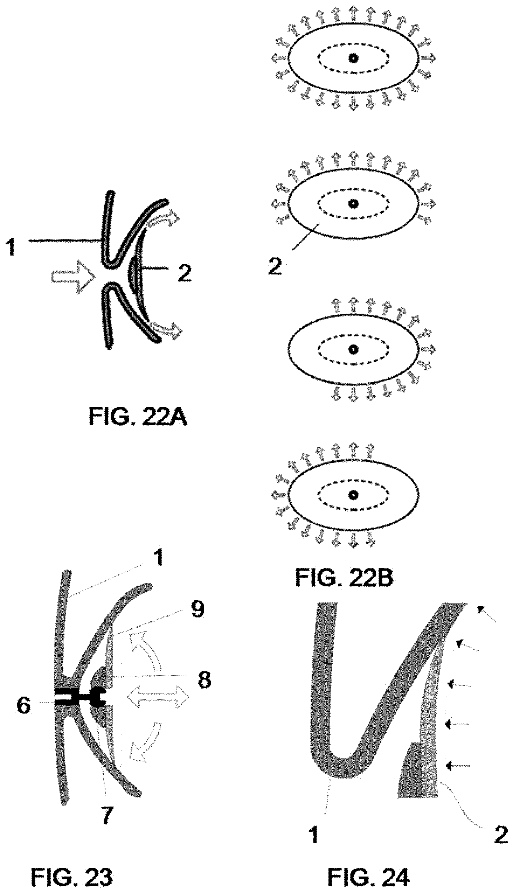

In another of the preferred examples of the invention, the valve body (1) conforms its geometry with a low relief (Fig. 24), so that the surface of the valve body (1) that is to be exposed to contact with the user's tongue is aligned with the surface of the valve flap (2), that will also be exposed to contact with the tongue, so that both elements generate a curved surface without jumps or ridges within the buccal cavity proper, which improves the contact of the buccal valve with the tongue and favours the functioning of the valve flap (2).

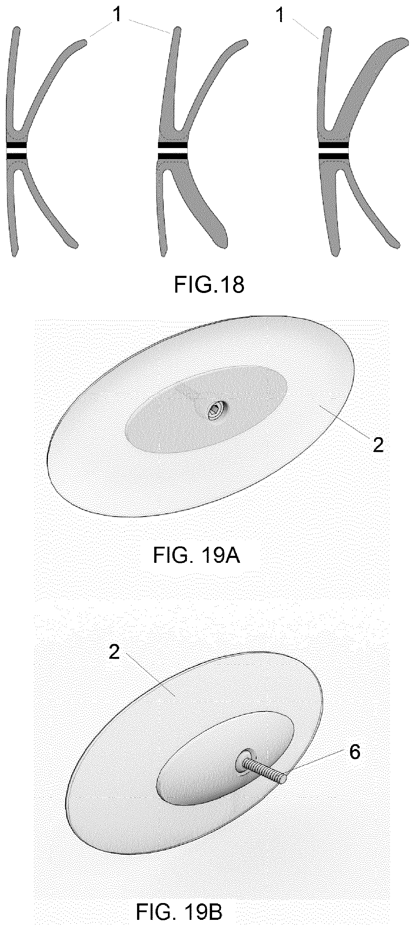

According to the invention, the valve body (1) may adapt the geometry of its cross-section in order to adapt it to the different positions of the user's dental arches by keeping a geometry that provides an adequate support for the contact with the valve flap (2) and the user's tongue (Fig. 18).

According to the invention, any of the variants of the valve body (1) may be made of a single material (single-material) or of several materials (multi-material), with a greater or lesser degree of flexibility, which provides the necessary physical qualities to said element to meet the purpose for which it has been designed.

In a preferred example of the invention, the valve body (1) may be made of two materials (bi-material) (Fig. 1), complementary to each other but of different physical features, so that one may be lighter and easier to shape, adequate to adapt to the varied geometry of the dental arches, and the other, more resistant to mechanical stress and more adequate to serve as an anchoring (6) to external elements of the valve body (1).

The valve body (1) of the invention described herein, in some of its variants, may also be conformed as a continuous and unitary object; meaning that none of its parts are mechanically assembled after manufacture, so that there is no structural joint that cuts the continuity of the material or materials with which it is made.

The shape and dimensions of the valve body (1) of the invention, in any of its variants, may be modified to be able to better adapt to the great variety of existing physiognomies, as long as it continues to meet the aforementioned functions attributed to the valve body (1) of the invention of buccal valve described herein.

Preferentially, the geometries of the valve body (1) of the various variants of the invention should be curved geometries that conform to the organic forms of the buccal anatomy and have no edges or angles that may damage the soft tissues of said anatomy.

The different variants of the valve body (1) (Fig.1-35) of the invention described herein may be made of any adequate material, including but not limited to metals, plastics, rubbers, ceramics, chromium, titanium and combinations thereof. Other materials may include acrylics, latex, polyethylene, polypropylene, polystyrene, polyvinyl chloride, polyvinylidene chloride, polyvinyl acetate, polyacrylate, styrene-butadiene copolymer, chlorinated polyethylene, polyvinylidene fluoride, ethylene vinyl acetate copolymer, ethylene vinyl acetate copolymer, vinyl acrylate-ethylene vinyl chloride copolymer, ethylene-vinyl acrylate acetate copolymer, vinyl chloride-ethylene vinyl acetate copolymer, nylon, acrylonitrile-butadiene copolymer, polyacrylonitrile, polyvinyl chloride, polychloroprene, polybutadiene, thermoplastic polyimide, polyacetal, polyphenylene sulfide, polycarbonate, thermoplastic polyurethane, thermoplastic resins, thermosetting resins, natural rubbers, synthetic rubbers (such as chloroprene rubber, styrene butadiene rubber, nitrile butadiene rubber, and copolymer of ethylene-propylene-diene terpolymer, silicone rubbers, fluorinated rubbers, and acrylic rubbers), elastomers (such as soft urethane or water-expanded polyurethane) and thermosetting resins (such as hard urethane, phenolic resins and melamine resins).

In a preferred example of the invention, biocompatible materials may be used, in particular in areas of the valve body (1) that are in direct contact with the user's body tissues. In addition to some of the materials described above, biocompatible materials may also include a biocompatible polymer and/or elastomeric material. Adequate biocompatible polymers may include materials such as: homopolymer and copolymers of vinyl acetate (such as ethylene vinyl acetate copolymer and polyvinyl chloride copolymer), alkylstyrene copolymers, homopolymer and acrylate copolymers (such as polypropylene, polymethylmethacrylate, polymethacrylate, polymethacrylate, ethylene glycol dimethacrylate, ethylene dimethacrylate, siloxane methacrylate and hydroxymethyl methacrylate, and the similar), polyvinylpyrrolidone, 2-pyrrolidone, polyacrylonitrile butadiene, polyamides, fluoropolymers (such as polytetrafluoroethylene, siloxane fluorine methacrylate and fluoride of polyvinyl), homopolymer and copolymers of styrene acrylonitrile, cellulose acetate, homopolymer and copolymers of acrylonitrile butadiene styrene, polymethylpentene, polysulfones polyimides, polyisobutylene, polymethylstyrene and other similar compounds belonging to the state of the art.

In the present invention, it is advantageous to use materials that are particularly flexible and elastic, which may be used as well as materials that are biocompatible and sterilisable, for example: medical plastics (such as acrylonitrile butadiene styrene (ABS), Latex, polypropylene, polycarbonate and polyetheretherketone), silicone, polyisoprene (PI) and other cross-linked elastomers or thermoplastic elastomers (TPE).

Other materials such as Teflon, Mylar, PFA, LDPE, Hytrel, HDPE and polyester may also be used in some parts of the buccal valve of the invention.

### - Valve flap (2):

The valve flap (2) is the other of the two constituent and defining elements of the buccal valve invention described herein.

As previously mentioned, the valve flap (2) of the invention described herein is used to simultaneously control the volume and direction of the airflow entering the buccal cavity proper, and reorient the incoming airflow in the buccal cavity proper, depending on the position of the user's tongue.

Also, the buccal valve of the invention described herein may be used to block the exit of the airflow during the expiratory phase by the air passage(s) passing through the valve body (1) in order to redirect said flow and generate positive expiratory pressure in the respiratory tract of the user.

According to the invention, the geometry, the mechanical capabilities of the materials with which it is manufactured, the type of anchoring (6) to be attached to the valve body (1), the connection between the elements thereof, and its position with respect to the buccal anatomy of the user within the buccal cavity proper, give the valve flap (2) the previously mentioned functional capabilities.

In this document, the term "valve flap proper" (2) refers to the more movable elements that conform the valve flap (2), such as an elastic membrane (9) and a membrane support (9), that may change the angle and position repeatedly.

In one of the preferred examples of the valve flap (2) of the invention described herein, the general geometry thereof may be seen in the form of a sun shade, where one can observe the anchoring arm (6) to the valve body (1) and the ball joint connection of the anchoring arm (6) with the valve flap proper and the flap proper.

The geometry and shape of the valve flap (2) of the invention may be very varied and change depending on the function to be performed, the material used to manufacture it and the buccal physiognomy of the user. Generally, curved forms or geometries are preferred, which adapt well to the organic geometry of the buccal anatomy, such as circular, elliptic, parabolic, hyperbolic, etc.

Some examples of these shapes may be seen in the figures of this document, where different sections of different valve flap variants (2) appear. These variants of the valve flap (2) are fundamentally shaped to better adapt to the different buccal physiognomies of the users, where the shape of the teeth, the type of bite and the dimensions of the dental arches will determine which of these variants is most adequate to correctly perform the functions attributed to the valve flap (2) of the invention.

In other examples of valve flap sections (2) described herein, it may be seen that the cross-sectional geometry of the flap proper is shaped in such a way that the membrane support (8) protects the membrane (9) of the flap (2), modifying the influence of the tongue on said membrane (9) to be able to reorient the airflow entering the buccal cavity proper.

Another figure of this document includes a drawing that shows how the valve flap of the invention described herein is placed with respect to the buccal anatomy of the user, connecting it to the valve body (1) and remaining installed in the interior of the buccal cavity proper, on the concave face of the dental arches and exposed to contact with the user's tongue. This allows it to control the direction of the airflow entering the buccal cavity proper, and reorient said flow into the buccal cavity proper, in terms of the position of the user's tongue. Depending on the position of the user, the tongue will adopt a position that will cause the valve flap (2) to react in one way or another, depending on the contact or non-contact thereof with the user's tongue.

As previously mentioned herein, the valve flap (2) of the invention is designed to orient the airflow entering the buccal cavity proper towards different areas of the buccal anatomy, such as the area below the tongue, the lateral area of the dental arches or the palatal area, in order to increase the level of turbulences of the flow and thus also prolong the exposure time of the incoming airflow to the moist tissues of the buccal cavity before reaching the pharyngeal area.

In another example of the present document it may be seen how the valve flap (2) can regulate the volume of airflow entering the buccal cavity proper through the adjustment of its anchoring system (6) with the valve body (1), which allows it to regulate the distance between the valve body (1) and the valve flap (2). Said distance influences the level of resistance offered by the valve flap (2) to the airflow entering the buccal cavity proper, decreasing or increasing the volume of airflow entering by the mouth to the respiratory system at a same level of effort for the user.

In a preferred example of the invention, the arm connecting the valve flap (2) with the valve body (1) is connected to the valve flap proper (2) through a ball joint (7). Said ball joint (7) allows the pressure exerted by the edge of the valve flap proper (2) on the contact surface with the valve body (1) to be more or less balanced along the entire perimeter of the edge thereof. That is to say that the rotation of the ball joint (7) in the three spatial directions allows the valve flap proper (2) to re-equilibrate the pressure exerted on the valve body (1) along its edge and better adapt to the different geometries of the valve body (1).

In a preferred example of the invention, it may be seen that the valve flap (2) may be made of three materials (tri-material), complementary to each other but of different physical features, that work consistently to improve their mechanical capacities and optimize their adaptability to the buccal anatomy. In said example, it is seen how a first structure, made of a more rigid material (adequate for making the anchoring arm (6)), is connected to a second structure (8), made of a slightly less rigid material (more adequate for connecting to the ball joint head (7) of the anchor arm (6)), which is connected to a third structure (9), made of a more flexible material (more adequate for performing the function of a flap membrane (9)). This improves the mechanical behaviour of the valve flap (2) and its capacity to adapt to the geometry of the valve body (1).

This balance of the pressure exerted by the valve flap (2) on the contact surface with the valve body (1) improves the functioning thereof and allows a shorter time of reaction to the change of direction of the respiratory flow between the inspiratory and the expiratory phases.

Moreover, the patellar connection between the previously mentioned elements of the valve flap (2), according to the invention, facilitates the adjustment of the distance between the valve body (1) and the valve flap proper (2), as the ball joint head (7) can be accessed from the face of the valve flap (2) leading to the inner part of the buccal cavity, and rotated with a wrench to regulate the previously mentioned distance.

In other of the preferred examples of the invention, the valve flap proper (2) has irregular surfaces (10) that help it to control the amount of contact surface existing between it and the valve body (1), thus preventing a possible excessive surface adhesion between said elements, generated by any type of liquid that could be interposed between both surfaces and that could affect the correct functioning of said valve flap (2).

In some cases, the irregular surface (10) of the valve flap (2) may also help said flap to better support the contacts with the user's tongue, increasing the structural inertia of the valve flap proper (2) in both parallel and perpendicular direction to the main plane thereof, but without interfering in the flexion movement towards the interior of the buccal cavity to be performed by said valve flap (2) in its usual functioning.

In other of the preferred examples of the invention, it may be seen that the surface of the valve flap (2) which is exposed to contact with the user's tongue, is aligned in one plane with the surface of the valve body (1) which is also exposed to contact with the tongue, and both create a surface without ridges or edges that improves the contact of the buccal valve with the tongue of the user making its use more comfortable.

Said alignment of the surfaces also improves the functioning of the valve flap (2) within the buccal cavity reducing the risk of interference between the user's tongue and the functioning of the valve flap (2).

In the examples of the invention described herein, the valve flap (2) may be temporarily connected to the valve body (1), so that if one valve flap (2) is damaged or does not work properly, it may be changed and replaced with another one in good condition.

In another possible example of the invention, the valve flap (2) may be permanently fixed to the valve body (1), so that the durability of both elements is linked, and when one of them is damaged or broken, the buccal valve has to be replaced in its entirety.

According to the invention described herein, the buccal valve may comprise one or several valve flaps that are fixed to its valve body (1) and modify the respiratory flow.

Likewise, according to the invention described herein, the valve flap (2) may comprise one or several parts of its geometry adequate to be either temporarily or permanently connected to the valve body (1), in order to stabilize itself in the buccal cavity proper and modify the respiratory flow.

According to the invention described herein, the type of anchoring (6) of the valve flap (2) with the valve body (1), as well as the type of connection between the different elements comprising it, may change depending on the functional requirements and the materials of which they are made. Such anchoring and types of connections include ball joints, joints, clamps, clips, welds, rings, fasteners, etc.

According to the invention described herein, the valve flap (2) may be made of only one material (single-material) or several materials (multi-material) that meet the requirements for its correct functioning and guarantee the durability thereof. For example, any of the materials previously mentioned in this publication may be used, as long as it meets the required safety and durability requirements.

### - Airflow resistance (3):

The airflow resistance (3) is one of the additional elements that are connected to the valve body (1) of the invention described herein and that give it new functional capabilities.

The buccal valve with airflow resistance (3) is another variant of the invention of the document described herein.

According to the invention, the airflow resistance (3) is characterized by working essentially in "flexion", collaborating with the soft tissues of the anatomy of the face, to give the buccal valve the capacity to increase the level of resistance offered to the exit of air from the buccal cavity during the expiratory phase.

According to the invention, the airflow resistance (3) depends on its anchoring to the valve body (1) and on said soft tissues to stabilize in the buccal cavity and be able to perform its function.

The airflow resistance (3) for the buccal valve of the invention described herein may be positioned within the buccal cavity, in the space between the soft tissues of the face anatomy and the dental arches, and in direct contact with the respiratory flow of the user, in order to increase the level of resistance offered to the passage thereof during the expiratory phase.

The airflow resistance (3) for the buccal valve of the invention described herein has as preferred objectives: not to interfere with the airflow entering the buccal cavity through the existing passages in the valve body (1) and provide resistance to the airflow exiting from the buccal cavity during the expiration phase.

Said airflow resistance (3) forces the outgoing airflow to pass through the contact surface that exists between the soft tissues of the face anatomy and the edge of its outer perimeter, so that the internal pressure in the respiratory tract must be increased to allow the airflow to pass through said surface and exit.

According to the invention, the geometry, the mechanical capabilities of the materials with which it is manufactured, its type of anchoring to the valve body (1) and its position within the buccal anatomy of the user, give the airflow resistance (3) of the valve the previously mentioned functional capabilities.

In this document the term "flap resistance proper" refers to the more flexible and movable elements that make up the airflow resistance (3), the resistance membrane and the membrane support.

In one of the preferred examples of the air flow resistance (3) of the invention described herein, we may see the general geometry thereof in the form of lobes with an airflow passage (11) therethrough. In such an example we may also observe its anchor arm to the valve body (1), the ball joint connection of the anchor arm to the flap resistance proper and the resistance membrane with its outer and inner edges as a fold.

In a preferred example of the invention, it may be seen that the airflow resistance (3) may be made of three materials (tri-material), complementary to each other but of different physical features, that work consistently to improve their mechanical capabilities and optimize their adaptability to the buccal anatomy. In said example, it is seen how a first structure, made of a more rigid material (adequate for making the anchoring arm), is connected with a second structure made of a slightly less rigid material (more adequate for connecting to the ball joint head of the anchor arm and absorb the deformations suffered by the flap resistance proper), which is connected to a third structure made of a more flexible material (suitable to be in contact with the soft tissues of the buccal anatomy). This improves the mechanical behaviour of the airflow resistance (3) of the valve and its capacity to adapt to the geometry of the buccal anatomy.

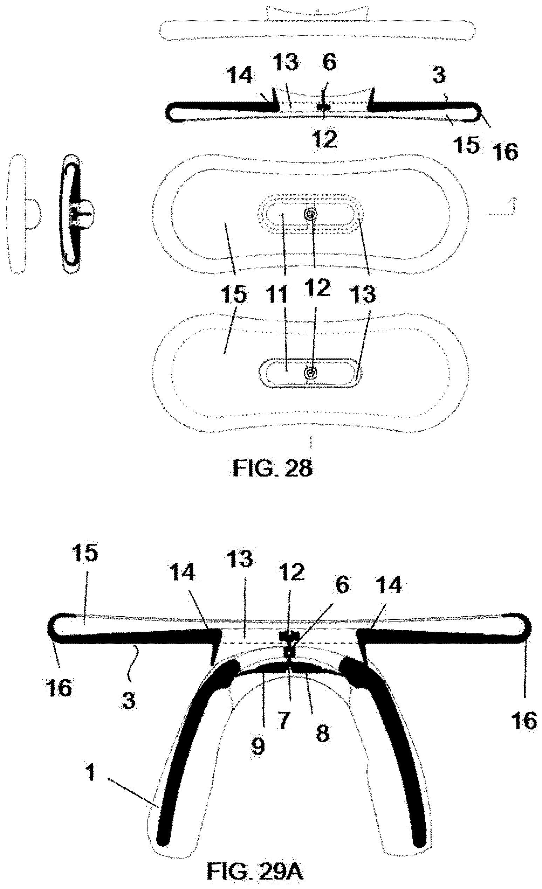

Figure 28 shows how the anchor arm (12), preferentially made of a rigid material, is connected to an airflow resistance membrane (13) made of a less rigid material, attached to a connecting support (14) between the valve body (1) and said resistance membrane (13), adequate to be connected to the ball joint (12), and where said resistance membrane (13) preferentially comprises an outer front edge (15) and an outer curved edge (16). In this way and as shown in the figure, the resistance membrane (13) is able to absorb the deformations suffered by the ends (16) of the airflow resistance element (3). This improves the mechanical behaviour of the airflow resistance element (3) of the valve and its capacity to adapt to the buccal anatomy.

The shape of the air flow resistance (3) of the invention may change depending on the functions to be performed, the material used for its manufacture and the user's buccal physiognomy. Generally, curved geometries are preferred which fit well into the buccal anatomy and tolerate the deformations to which this flow resistance is subjected in order to function correctly.

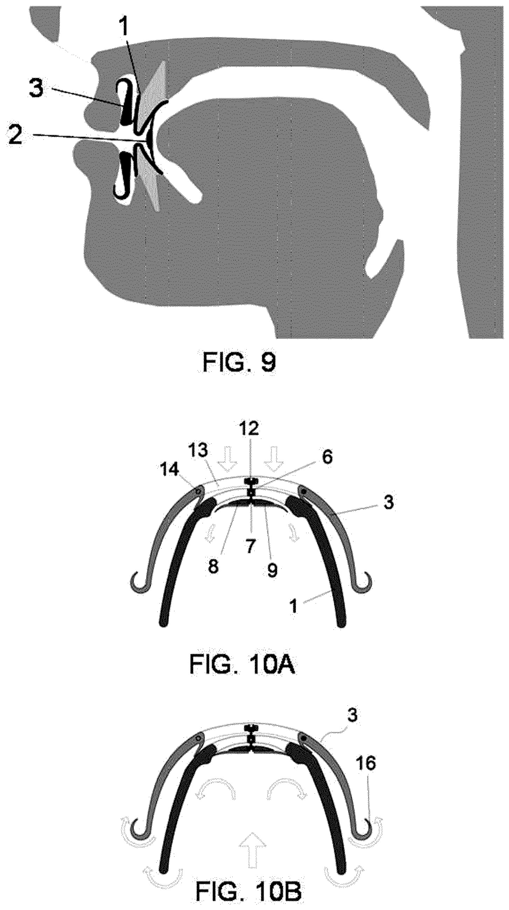

In the figures of this document (e.g. Figs 1-2) we may see the position of the flap proper (2), of the airflow resistance (3) when it is not installed in the buccal cavity, and the position of the same flap when it is installed in the buccal cavity (Fig. 9). In these diagrams, it is possible to see the temporary deformation which the airflow resistance flap of the invention must suffer to be installed in the buccal cavity and be able to correctly perform its airflow resistance function (3), working in collaboration with the soft tissues of the user's face anatomy.

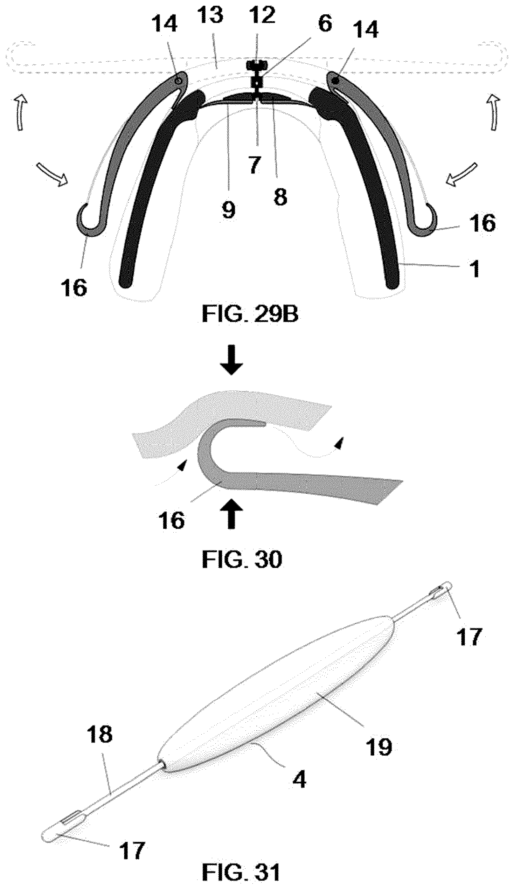

In other figure of this document (Fig. 30), it may be seen how the outer curved edge (16) of the airflow resistance membrane (3) of the invention exerts a force against the soft tissues of the face anatomy, generated by the natural tendency of the flap resistance to recover its initial position before being installed inside the buccal cavity. Said soft and elastic tissues of the buccal anatomy, in reaction to the force acting on them, exert an equal and opposite force on the edge of the flap resistance, so that the contact surface between them increases and bears a pressure that hinders the passage of airflow through it; this pressure, generated by the forces confronted, helps to increase the internal pressure within the respiratory tract.

In a preferred example of the invention, the pressure exerted by the outer curved edge of the flow resistance membrane on the soft tissues of the face anatomy is continuous and more or less balanced along its entire perimeter, due to the particularity of its curved geometry, the physical features of the materials composing it and the ball joint anchoring between said flap resistance proper and its connecting arm with the valve body (1).

The ball joint anchoring between the flap resistance proper and the anchoring arm with the valve body (1), according to the invention, allows a more balanced distribution of the pressure exerted by the outer edge of the flap resistance on the soft tissues of the anatomy and the pressure exerted by the inner edge thereof on the valve body (1).

Said ball joint connection of the component elements, together with the anchoring arm to the valve body (1) allows the airflow resistance (3) to regulate the angle and distance between the flap resistance and the valve body (1), improving the adaptation of the airflow resistance (3) to the different buccal physiognomies and the geometry of the valve body (1).

In other preferred example of the invention, it is seen how the curved geometry of the outer edge of the flap resistance proper improves the contact with the soft tissues of the buccal anatomy, rebalancing the pressure exerted on said tissues; redirects the expiratory airflow, so that it exits between said edge and the soft tissue of the buccal anatomy, and prevents the return of the airflow into the interior of the buccal cavity.

In the same preferred example of the invention we may see how the curved geometry of the inner edge in the flap resistance proper, in relation to the airflow passage therethrough, absorbs the deformation thereof when it is installed in the buccal cavity to adapt it to the geometry of the valve body (1), in order to seal and prevent the passage of the airflow through that area. In this way, all the expiratory airflow is forced out surrounding the outer edge of the flap resistance proper.

According to a preferred example of the invention, the force exerted by the outer edge of the flap resistance on the soft tissues of the face anatomy may also help to open the orbicularis muscles of the face, facilitating the passage of the airflow entering the buccal cavity through the valve body (1).

According to the invention described herein, an airflow resistance (3) is configured to prevent the entry of airflow into the buccal cavity proper, except for the air passage or passages provided for this purpose, going through the valve body (1) of the invention.

In the examples of the invention described herein, the airflow resistance (3) may be temporarily connected to the valve body (1), so that if a valve resistance is damaged or does not function correctly it may be changed and replaced it with another one in good condition.

In another possible example of the invention, the airflow resistance (3) of the valve may be permanently fixed to the valve body (1) becoming a single object, so that the durability of both elements is linked, and when one of the two is damaged or broken, the buccal valve must be replaced in its entirety.

The buccal valve, according to the invention described herein, may comprise one or several airflow resistances that are fixed to its valve body (1) and may modify the respiratory flow.

Likewise, according to the invention described herein, the airflow resistance (3) of the valve may comprise one or more parts of its geometry adequate to be connected with the valve body (1) of the invention, either temporarily or permanently, in order to stabilize in the buccal cavity proper and modify the respiratory flow.

According to the invention described herein, the type of anchoring of the valve's airflow resistance (3) with the valve body (1), as well as the type of connection between the different elements that comprise it, may change depending on the functional requirements and the materials of which they are made. Such anchorings and types of connections comprise ball joints, joints, clamps, clips, welds, rings, fasteners, etc.

The airflow resistance (3) of the valve, according to the invention described herein, may be made of a single material (single-material) or several materials (multi-material) that meet the requirements for its correct functioning and guarantee the durability of the same. For example, any of the materials previously mentioned in this document may be used, as long as it meets the required safety and durability requirements.

### - Tongue movement controller (4):

In the invention described herein, the tongue movement controller (4) is another one of the additional elements that are connected to the valve body (1) and give the buccal valve a series of new functional capabilities.

The buccal valve with tongue movement controller (4) is another variant of the invention described herein.

According to the invention, the tongue movement controller (4) is characterized by working essentially in flexion to perform its function of partially controlling the movement of the tongue of the user during the moments when the tongue muscles are relaxed and the tongue is moved by the effect of the force of gravity.

According to the invention, the tongue movement controller (4) depends on its anchoring (17) to the ends of the valve body (1) to stabilize itself in the buccal cavity and be able to perform its function.

The tongue movement controller (4) for the buccal valve of the invention described herein may be positioned within the buccal cavity proper, passing through it and connecting the ends of the valve body (1), so that it remains in direct contact with the user's tongue to partially control its movements.

The main purpose of the tongue movement controller (4) for the buccal valve of the invention described herein is to prevent, when necessary, the tongue from moving excessively towards the palatine and the glossopharyngeal areas when it is relaxed, which would collapse the airflow passage during the inspiratory phase.

The tongue movement controller (4) stabilizes and controls the movements of the tongue by placing a portion of the controller on the top surface of the tongue, and exerting a slight pressure thereon to increase the contact surface between them, limiting the movements when the tongue relaxes.

Its geometry, the mechanical capabilities of the materials with which it is made, the type of anchoring (17) with which it is fixed to the valve body (1) and its position within the buccal anatomy of the user, give the tongue movement controller (4) of the valve the previously mentioned functional capabilities according to the invention.

In one of the preferred examples of the tongue movement controller (4) of the invention described herein (Figs. 31-34), it is possible to observe the general geometry of the same in the shape of a longitudinal structure, with part of its geometry shaped as a superficial support element (19). This example also shows the anchorings (17) of the tongue movement controller to the valve body (1), the main longitudinal cylinder-shaped structure (18) connecting said anchorings (17) and the support element (19) covering the main structure (18).

The shape of the tongue movement controller (4) of the invention may change depending on the functions to be performed, the material used for its manufacture and the user's buccal physiognomy. Curved geometries are generally preferred, which adapt well to the buccal anatomy and tolerate the deformations to which said tongue movement controller (4) is subjected in order to function correctly.

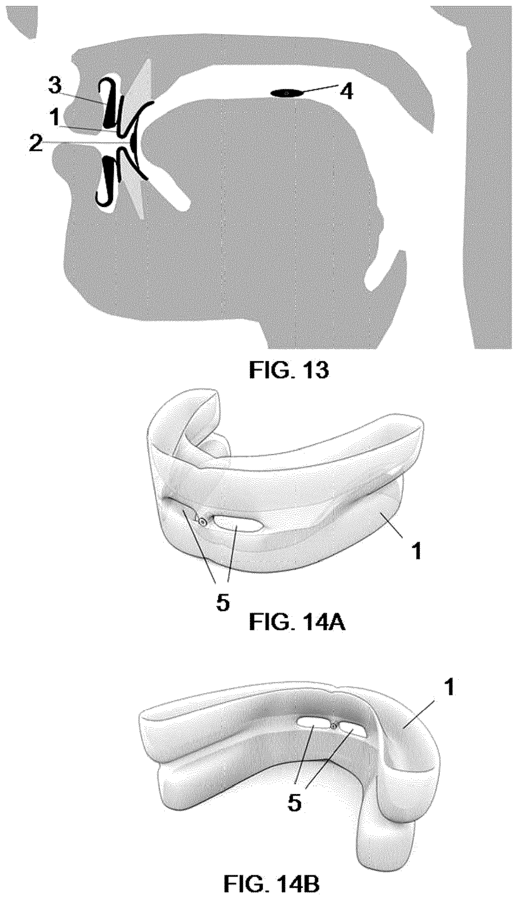

The figures in this document (Figures 31-34) show the position of the tongue movement controller (4) when it is not installed in the buccal cavity and its position when it is installed inside the buccal cavity (Fig. 13). In this scheme, it is possible to see the temporary deformation that the tongue movement controller (4) of the invention must undergo to be installed in the buccal cavity to correctly perform its function of controlling the movement of the tongue.

Other figures of this document (Figures 31-32) show how the support element (19) of the tongue movement controller (4) of the invention covers the main longitudinal structure (18) without adhering thereto, so that said support element (19) allows a free relative rotation of the longitudinal structure (18) within the same, so as not to interfere with the deformation suffered by the longitudinal structure when it is installed in the buccal cavity. That is, the support element (19) does not generate internal forces (torsion, traction, compression) within the longitudinal structure when it is deformed to adapt to the anatomy of the user's tongue.

This patellar connection between the two elements makes it possible that the longitudinal structure can deform freely, optimizing its mechanical capabilities; that the pressure exerted by said longitudinal structure on the tongue is balanced along its entire length; and that the tongue does not suffer cutting forces, only perpendicular ones generated by the deformation of the longitudinal structure, given that said cutting forces are not transferred from the longitudinal structure to the support element (19) that interposes between the structure and the tongue.

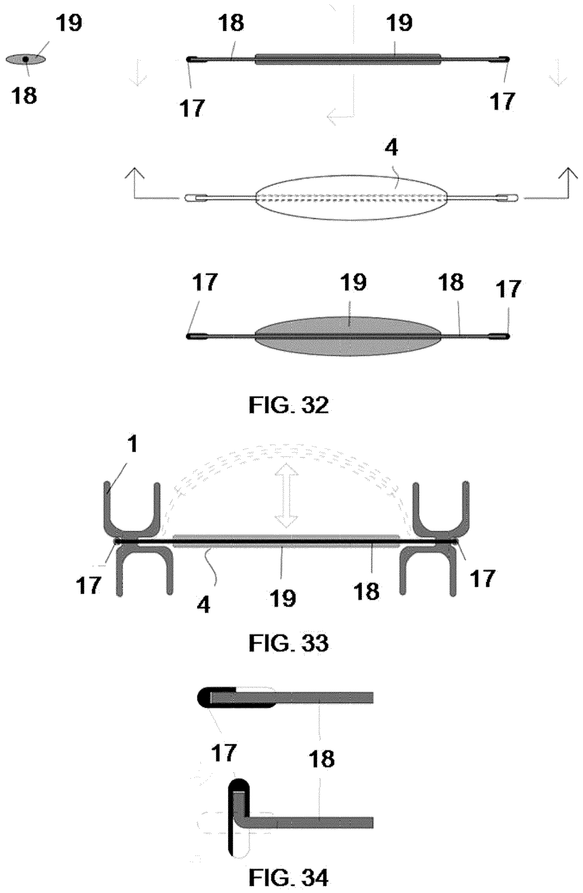

Another figure of this document (Fig. 34) shows how the anchoring element (17) of the tongue movement controller (4) of the invention is anchored to the valve body (1), changing its angle with respect to the longitudinal structure of the controller.

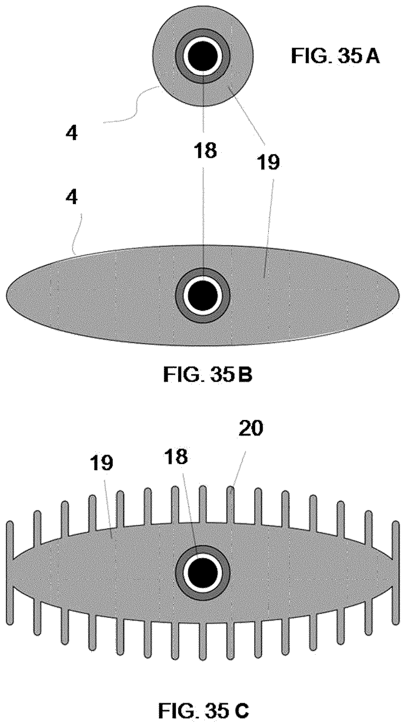

According to the invention, in the Figures 35A-35C of this document it is possible to see the different geometries preferentially adopted by the cross-section of the support element (19) of the tongue movement controller (4). Said geometries are preferentially curves, and suitable to better absorb the deformations and better adapt to the buccal anatomy. One of these sections (Fig. 35C) shows how the support element (19) comprises a series of irregular surfaces (20) that help to improve the contact surface of this element with the tongue and drain possible liquids that could interpose between the two surfaces.

In the examples of the invention described herein, the tongue movement controller (4) may be temporarily connected to the valve body (1), so that, if said element is damaged or does not work properly, it may be changed and replaced with another one in good condition.

In another possible example of the invention, the tongue movement controller (4) of the valve may be permanently fixed to the valve body (1) so that the durability of both elements is linked, in which case when one of the two is damaged or broken, it is necessary to replace the buccal valve in its entirety.

According to the invention described herein, the buccal valve may comprise one or several tongue movement controllers (4) that are fixed to its valve body (1) and may facilitate the respiratory flow.

Likewise, the tongue movement controller (4) of the valve, according to the invention described herein, may comprise one or several parts of its geometry, suitable to be either temporarily or permanently connected with the valve body (1), in order to stabilize it in the buccal cavity and facilitate the respiratory flow.

According to the invention described herein, the type of anchoring (17) of the tongue movement controller (4) with the valve body (1), as well as the type of connection between the different elements comprising it, may change depending on the functional requirements and the materials of which they are made. Such anchorings and types of connections comprise ball joints, joints, clamps, clips, welds, rings, cannulas, clasps, etc.

The air flow resistance (3) of the valve, according to the invention described herein, may be made of a single material (single-material) or several materials (multi-material) that meet the requirements for proper functioning and guarantee the durability of the same. For example, any of the materials previously mentioned in this publication may be used, as long as it meets the required safety and durability requirements.

### - Manufacture of the buccal valve:

The various elements comprising the buccal valve of the invention described herein may be elaborated through different industrial processes (immersion, injection, centrifugation, mechanical polishing, chemical polishing, material addition, material subtraction, stereolithography, laser sintering, electron beam melting, abrasion, pulverization, vaporization, priming, turnery, thermoforming, extrusion, 3D printing, etc.), depending on the nature of the material with which it is manufactured, the complexity of its geometry and the required functional requirements. Any of the materials hereinbefore mentioned may be used for the manufacture of the buccal valve of the invention described herein.

For example, any of the variants of the elements of the valve flap proper (2), the flow resistance flap proper and the support element (19) of the tongue movement controller (4) are configured in such a way that industrial techniques of melding by immersion, injection or 3D printing may be the most adequate for their manufacture. Also, the longitudinal structure of the tongue movement controller (4) and the valve body (1) for the buccal valve of the invention may be made with these procedures.

For example, for the manufacture of the anchoring elements of the different components of the buccal valve of the invention (anchor arms, ball joint heads, bolts and threaded tubes), given the previously mentioned features, the addition of material, such as laser sintering or electron beam melting techniques could be more appropriate. However, any of the industrial procedures necessary to attribute to the buccal valve elements the required functional features may be used.

In case the elements are made by means of immersion processes in dispersions, solutions or masses in a viscose state (e.g. nets or liquid polymers), the different thicknesses of the layers of material may be achieved by means of immersion moulds with varied geometries. For example, the reduction or thickening of section in the material may be achieved by immersing moulds with ridges that have small radii of curvature. The superficial tension of the material of dissolution, together with the geometry and temperature of the mould used, may generate sections of material of variable curvature and thickness. These moulds may be designed to be "lost" moulds, i.e., they may be permanently imbued in the material of dissolution after having been applied, becoming an internal substructure of the elements of the buccal valve. In case the elements are manufactured by means of injection moulding or compression moulding processes, the reductions, thickenings and different superficial textures may be carried out by machining in the mould, both in the male and in the female.

In the case of the valve body (1) of the buccal valve of the invention described herein, there are two different methods to produce said element: one is the standardized production of the element, and the other is the personalized production for each user.

The fundamental difference between the two methods does not have to be the manufacturing process itself, but rather the prior process of compilation of information on the buccal anatomy of the user before starting the manufacture of the object.

The manufacturing processes in either of the two methods of production could be any of the industrialized or manual processes previously mentioned in this document.

With the first method, a series of standardized models of valve bodies of different sizes would be produced, based on statistical studies of the shapes of the different buccal anatomies, that would be approximately adapted to the dental arches of most of the possible users, and that subsequently, the user or the corresponding medical entity could customize to the exact anatomy of each person, using a second material that would be interposed between the structure of the standardized model and the user's dental arch, that would fit the exact shape of the same, due to the physical features of said material.

The second method includes a phase, prior to the manufacture, where information is obtained on the shape of the buccal cavity, the features of the dental arches and the final position that is sought between both dental arches and the type of bite of the user, which will later be used during the manufacturing phase.

The compilation of this information may be done in a standard manner with dental impressions, bite templates, moulds, manual scanning, etc., or through systems that generate information on the buccal anatomy of the user on a digital basis, such as computed tomography, scanning, conical beam computed tomography or digital photogrammetry.

These digital information compilation systems typically create STL (Standard Triangle Language) files that are often used by material adding manufacturing element machines, such as 3D printing or laser sintering.

Some manufacturing by subtraction of material systems, such as industrial turnery, may also use this type of digital files to create their objects.

This digital information allows the creation of a personalized buccal valve for a remote user, only requiring the presence of the user at the time of the initial data collection.

The usual method for compilation of data of the user's anatomy is firstly to define, together with the user, the exact position that the lower dental arch will adopt with respect to the upper dental arch when the valve body (1) is installed within the buccal cavity.

To determine this position, an interdental spacer, such as a George Gauge, may be used, which determines the separation between the two dental arches, the advance of one vis-à-vis the other, and the bite between the two.

Secondly, and once the desired position of the jaw is defined, a scan, a tomography or digital photos of the buccal anatomy are made to generate a file in digital format (e.g. STL) that can serve as a base to create the desired valve body (1). Dental impressions may also be made to generate moulds, as well as a study of the bite through templates or of the same dental spacer.

In some cases CBCT scanning equipment may be used to perform mould scans made with dental impressions to generate the STL computer file.

Thirdly, all this information serves as a basis to be able to manufacture a customized buccal valve for each user in the laboratory.

Finally, the method may include the cutting and/or polishing of the material used to create said object, to ensure that it is comfortable to use prior to installing the buccal valve for an extended period of time in the buccal cavity of the user. In particular, it must be verified that the valve body (1) does not oscillate or lift, that it does not rub the gums, that it is not too tight and that it does not cause pain to the teeth, the muscles or the articulation of the jaw. This process usually also includes a follow-up questionnaire, sleep study and orofacial pain examination.

In the case of the elements of the invention that are manufactured by the polymer injection method, the process may require several moulds, male and female, that configure the external and internal geometry of the element and that should be manufactured in a way that allows the removal thereof after the element has been injected.

In some of the preferred examples of the invention described herein, the buccal valve elements may be fixed together by means of a chemical or mechanical welding that may use ultraviolet light or ultrasound.

Certain parts of the elements that make up the buccal valve of the invention described herein may be made by cutting with blades or laser light on previously shaped forms.

According to the invention, the anchoring of certain parts with others may also be carried out by the application of mechanical force that causes one part to fit another part, such as the ball joint head with the elements of the different valve flaps, and of the airflow resistance (3).

The invention provides a buccal valve that allows controlling the airflow both in and out of the respiratory cavities of the user, in a simple way so that the user him/herself can comfortably install and uninstall it from his/her buccal cavity using his/her own hands. Additionally, the buccal valve of the invention may be combined with other devices, although it does not need an external source of energy for proper functioning.

The capacity to be installed in the buccal cavity of the user to simultaneously control the volume and direction of the airflow entering the buccal cavity (once it has passed through the dental arches), reorient said flow into the buccal cavity depending on the position of the tongue of the user, and work together with the soft tissues of the buccal anatomy to generate positive expiratory pressure in the airway, is what characterizes the buccal valve of the invention described herein, in its most basic version, with respect to other buccal valves of the state of the art.

The positive expiratory pressure generated by the buccal valve of the invention may be oriented towards the medical treatment of respiratory disorders, as well as non-medical purposes, such as sports training and performance of activities with aerobic requirements.

The present invention may comprise a series of additional elements that help to increase the level of positive expiratory pressure generated by the buccal valve in the respiratory tract of the user and to control the movement of the tongue within the buccal cavity.

In another preferred example of the invention, the buccal valve may be combined with a toroidal nasal device or other treatments to modify the airflow entering the respiratory cavity and control the percentage of air entering by both the mouth and the nose, and its resulting combination, thus helping to treat respiratory diseases and disorders and/or support non-therapeutic uses such as sports training or activities with aerobic needs. In summary, in this preferred example of the invention the main objective is not to maximize by all means the airflow entering through the buccal cavity into the respiratory system of the user, since this is a secondary objective. The main purpose of the buccal valve in this example is to control the percentage of airflow entering the buccal cavity with respect to the percentage entering the nasal cavity so as to find an optimal balance between both.

## Claims

1. Buccal valve, suitable for modifying the respiratory flow of a user, comprising:
- a valve body (1), adapted to be arranged between the dental arches of the user, wherein said valve body (1) comprises one or more airflow passages (5) therethrough; and
- at least one valve flap (2) configured to be connected to the valve body (1);
wherein the valve body (1) and the valve flap (2) are arranged so that, when the buccal valve is installed in the buccal cavity of the user, the at least one valve flap (2) is positioned in the buccal cavity proper and exposed to direct contact with the tongue of the user and wherein the valve flap is adapted to regulate the flow rate and direction of the airflow entering the buccal cavity proper through the airflow passages; and **characterized in that** the valve flap (2) is connected to the valve body (1) by means of an anchoring arm (6) and a ball joint (7).

2. Buccal valve, according to the preceding claim, wherein the connection between the valve flap (2) and the valve body (1) allows the modification of the distance between the two.

3. Buccal valve, according to any of the preceding claims, wherein the ball joint (7) is comprised in the valve flap (2) and/or the valve body (1).

4. Buccal valve, according to any of the preceding claims, wherein the valve flap (2) is detachable from the valve body (1).

5. Buccal valve, according to any of the preceding claims, wherein the surfaces of the valve flap (2) and the valve body (1) that are exposed to contact with the tongue of the user are aligned in a same curved plane.

6. Buccal valve, according to any of the preceding claims, comprising one or more airflow resistance elements (3), adapted for their connection to the valve body (1) and arranged so that, in collaboration with the soft tissues of the face anatomy when the valve is in use, they allow to increase the level of resistance offered at the exit of the expiratory flow from the buccal cavity.

7. Buccal valve, according to claim 6, wherein the connection between the airflow resistance element (3) and the valve body (1) allows the modification of the distance between the two.

8. Buccal valve according to claims 6-7, wherein the airflow resistance element (3) comprises a ball joint (12).

9. Buccal valve, according to claims 6-8, wherein the airflow resistance element (3) is detachable from the valve body (1).

10. Buccal valve, according to claims 6-9, wherein at least one of the elements forming the airflow resistance (3) is adapted to be positioned in the space between the dental arches and the soft tissues of the user's face anatomy.

11. Buccal valve, according to any of the preceding claims, further comprising one or more tongue movement control elements (4) configured to be connected to the valve body (1) and adapted to be disposed in the buccal cavity proper, exposed to contact with the tongue of the user, so as to allow partial control of the movements of the tongue of said user when the valve is in use.

12. Buccal valve, according to claim 11, that comprises at least one main longitudinal structure (18) and a support element (19) that allows the free relative rotation of said main longitudinal structure (18) therein by means of a ball joint-type connection.

13. Buccal valve, according to any of claims 11-12, wherein the tongue movement controller (4) comprises an anchoring (17) of said tongue movement controller (4) to the valve body (1) by means of one or more ball joints, joints, clamps, clips, welds, rings, cannulas and/or clasps.

14. Buccal valve, according to any of the preceding claims, wherein at least the valve flap (2) and/or the valve body (1) are made of a flexible material, and/or wherein at least a part of the manufacturing materials of said valve are biocompatible and/or biodegradable.

## Patentansprüche

1. Bukkalklappe, geeignet zur Modifizierung des Atemflusses eines Benutzers, umfassend:
- einen Ventilkörper (1), der zum Anordnen zwischen Zahnbögen des Benutzers ausgelegt ist, wobei der Ventilkörper (1) eine oder mehrere Luftdurchgangskanäle (5) dadurch umfasst; und
- mindestens eine Ventilklappe (2), die zum Verbinden mit dem Ventilkörper (1) konfiguriert ist;
wobei der Ventilkörper (1) und die Ventilklappe (2) so angeordnet sind, dass, wenn die Bukkalklappe in der bukkalen Höhle des Benutzers installiert ist, die mindestens eine Ventilklappe (2) in der eigentlichen bukkalen Höhle positioniert ist und einem direkten Kontakt mit der Zunge des Benutzers ausgesetzt ist und wobei die Ventilklappe dazu ausgelegt ist, um die Durchflussrate und Richtung des Luftflusses zu regulieren, der durch die Luftdurchgangskanäle in die bukkale Höhle eintritt;
und **dadurch gekennzeichnet, dass** die Ventilklappe (2) mit dem Ventilkörper (1) mittels eines Verankerungsarms (6) und eines Kugelgelenks (7) verbunden ist.

2. Bukkalklappe nach dem vorhergehenden Anspruch, wobei die Verbindung zwischen der Ventilklappe (2) und dem Ventilkörper (1) die Veränderung des Abstands zwischen den beiden zulässt.

3. Bukkalklappe nach einem der vorhergehenden Ansprüche, wobei das Kugelgelenk (7) in der Ventilklappe (2) und/oder dem Ventilkörper (1) umfasst ist.

4. Bukkalklappe nach einem der vorhergehenden Ansprüche, wobei die Ventilklappe (2) von dem Ventilkörper (1) abnehmbar ist.

5. Bukkalklappe nach einem der vorhergehenden Ansprüche, wobei die Oberflächen der Ventilklappe (2) und des Ventilkörpers (1), die dem Kontakt mit der Zunge des Benutzers ausgesetzt sind, auf einer gleichen gekrümmten Ebene ausgerichtet sind.

6. Bukkalklappe nach einem der vorhergehenden Ansprüche, umfassend einen oder mehrere Luftdurchfluss-Widerstandselemente (3), die zu ihrer Verbindung mit dem Ventilkörper (1) ausgelegt sind und derart angeordnet sind, dass sie in Zusammenwirkung mit den Weichgeweben der Gesichtsanatomie bei Gebrauch der Klappe zulassen, das Widerstandsniveau, das am Austritt des Atemflusses aus der bukkalen Höhle geboten wird, zu erhöhen.

7. Bukkalklappe nach Anspruch 6, wobei die Verbindung zwischen dem Luftdurchfluss-Widerstandselement (3) und dem Ventilkörper (1) die Veränderung des Abstands zwischen den beiden zulässt.

8. Bukkalklappe nach einem der Ansprüche 6 bis 7, wobei das Luftdurchfluss-Widerstandselement (3) ein Kugelgelenk (12) umfasst.

9. Bukkalklappe nach einem der Ansprüche 6 bis 8, wobei das Luftdurchfluss-Widerstandselement (3) von dem Ventilkörper (1) abnehmbar ist.

10. Bukkalklappe nach einem der Ansprüche 6 bis 9, wobei mindestens eines der Elemente, das den Luftdurchfluss-Widerstand (3) bildet, dazu ausgelegt ist, um in dem Raum zwischen den Zahnbögen und den Weichgeweben der Gesichtsanatomie des Benutzers positioniert zu werden.

11. Bukkalklappe nach einem der vorhergehenden Ansprüche, ferner umfassend eines oder mehrere Steuerelemente der Zungenbewegung (4), die zum Verbinden mit dem Ventilkörper (1) konfiguriert sind und ausgelegt sind, um in der bukkalen Höhle selbst angeordnet zu werden, die dem Kontakt mit der Zunge des Benutzers ausgesetzt sind, um eine teilweise Kontrolle der Zungenbewegungen des Benutzers zu ermöglichen, wenn die Klappe in Betrieb ist.

12. Bukkalklappe nach Anspruch 11, die mindestens eine Hauptlängsstruktur (18) und ein Abstützelement (19) umfasst, das eine freie relative Drehung der Hauptlängsstruktur (18) darin mittels einer kugelgelenkartigen Verbindung ermöglicht.

13. Bukkalklappe nach einem der Ansprüche 11 bis 12, wobei die Zungenbewegungssteuerung (4) eine Verankerung (17) der Zungenbewegungssteuerung (4) in dem Ventilkörper (1) mittels einer oder mehrerer Kugelgelenke, Verbindungen, Klemmen, Clips, Schweißnähte, Ringe, Kanülen und/oder Spangen umfasst.

14. Bukkalklappe nach einem der vorhergehenden Ansprüche, wobei mindestens die Ventilklappe (2) und/oder der Ventilkörper (1) aus einem flexiblen Material hergestellt sind und/oder wobei mindestens ein Teil der Herstellungsmaterialien der Klappe biokompatibel und/oder biologisch abbaubar ist.

## Revendications

1. Valve buccale, adaptée pour modifier le flux respiratoire d'un utilisateur, comprenant :
- un corps de valve (1), adapté pour être disposé entre les arcades dentaires de l'utilisateur, dans laquelle ledit corps de valve (1) comprend un ou plusieurs passages de flux d'air (5) à travers celui-ci ; et
- au moins un clapet de valve (2) configuré pour être raccordé au corps de valve (1) ;
dans laquelle le corps de valve (1) et le clapet de valve (2) sont disposés de sorte que, lorsque la valve buccale est installée dans la cavité buccale de l'utilisateur, l'au moins un clapet de valve (2) est positionné dans la cavité buccale proprement dite et exposé au contact direct avec la langue de l'utilisateur et dans laquelle le clapet de valve est adapté pour réguler le débit et la direction du flux d'air entrant dans la cavité buccale proprement dite à travers les passages de flux d'air ;
et **caractérisée en ce que** le clapet de valve (2) est raccordé au corps de valve (1) au moyen d'un bras d'ancrage (6) et d'un joint à rotule (7).

2. Valve buccale, selon la revendication précédente, dans laquelle le raccordement entre le clapet de valve (2) et le corps de valve (1) permet la modification de la distance entre les deux.

3. Valve buccale, selon l'une quelconque des revendications précédentes, dans laquelle le joint à rotule (7) est compris dans le clapet de valve (2) et/ou le corps de valve (1).

4. Valve buccale, selon l'une quelconque des revendications précédentes, dans laquelle le clapet de valve (2) est détachable du corps de valve (1).

5. Valve buccale, selon l'une quelconque des revendications précédentes, dans laquelle les surfaces du clapet de valve (2) et du corps de valve (1) qui sont exposées au contact avec la langue de l'utilisateur sont alignées dans un même plan incurvé.

6. Valve buccale, selon l'une quelconque des revendications précédentes, comprenant un ou plusieurs éléments de résistance au flux d'air (3), adaptés pour leur raccordement au corps de valve (1) et disposés de sorte qu'ils permettent d'augmenter, en collaboration avec les tissus mous de l'anatomie du visage lorsque la valve est utilisée, le niveau de résistance offert à la sortie du flux expiratoire de la cavité buccale.

7. Valve buccale, selon la revendication 6, dans laquelle le raccordement entre l'élément de résistance au flux d'air (3) et le corps de valve (1) permet la modification de la distance entre les deux.

8. Valve buccale selon les revendications 6 à 7, dans laquelle l'élément de résistance au flux d'air (3) comprend un joint à rotule (12).

9. Valve buccale, selon les revendications 6 à 8, dans laquelle l'élément de résistance au flux d'air (3) est détachable du corps de valve (1).

10. Valve buccale, selon les revendications 6 à 9, dans laquelle au moins l'un des éléments formant la résistance de flux d'air (3) est adapté pour être positionné dans l'espace entre les arcades dentaires et les tissus mous de l'anatomie du visage de l'utilisateur.

11. Valve buccale, selon l'une quelconque des revendications précédentes, comprenant en outre un ou plusieurs éléments de contrôle de mouvement de la langue (4) configurés pour être raccordés au corps de valve (1) et adaptés pour être disposés dans la cavité buccale proprement dite, exposée au contact avec la langue de l'utilisateur, de sorte à permettre un contrôle partiel des mouvements de la langue dudit utilisateur lorsque la valve est utilisée.

12. Valve buccale, selon la revendication 11, qui comprend au moins une structure longitudinale principale (18) et un élément de support (19) qui permet la rotation relative libre de ladite structure longitudinale principale (18) à l'intérieur au moyen d'un raccordement de type joint à rotule.

13. Valve buccale, selon l'une quelconque des revendications 11 à 12, dans laquelle le contrôleur de mouvement de la langue (4) comprend un ancrage (17) dudit contrôleur de mouvement de la langue (4) au corps de valve (1) au moyen d'un ou plusieurs joints à rotule, joints, pinces, clips, soudures, anneaux, canules et/ou fermoirs.

14. Valve buccale, selon l'une quelconque des revendications précédentes, dans laquelle au moins le clapet de valve (2) et/ou le corps de valve (1) sont constitués d'un matériau flexible, et/ou dans laquelle au moins une partie des matériaux de fabrication de ladite valve sont biocompatibles et/ou biodégradables.
